# EUROPEAN PATENT APPLICATION

(11) **EP 4 741 056 A1**
(43) Date of publication of application: **13.05.2026**
(21) Application number: 24211263.9
(22) Date of filing: 06.11.2024
(51) Int. Cl.: B01L 3/00, G01N 35/00, G01N 33/49, G01N 33/86

(54) **METHOD FOR PROCESSING A BIOLOGICAL SAMPLE AND DEVICE THEREFOR**

(71) Applicant: Beckman Coulter, Inc., Brea, CA 92821 (US)
(72) Inventor: Naetzer, Beate, 85604 Zorneding (DE); Mael, Eric, Brea, CA 92821 (US); Benegal, Aditya, 560055 Bangalore (IN)
(74) Representative: Paustian & Partner Patentanwälte mbB

(57) **Abstract**

A first aspect of the present disclosure is related to a method for processing a biological sample with an automated laboratory system,
comprising the steps:
- loading a primary tube containing a primary biological sample comprising a pre-defined agent, e.g. Platelets;
- performing a centrifugation of the primary biological sample;
- transferring at least a part of the primary biological sample as a secondary biological sample into a secondary tube ;
- performing a centrifugation of the secondary biological sample ;
- providing a sample based on the centrifuged secondary biological sample.

## Description

### Technical field

This disclosure relates to methods for processing a biological sample with an automated laboratory system and to automated laboratory systems.

### Background

In a laboratory, centrifugation is used to separate all kinds of biological samples from one or more agents. The presence of the latter can spoil an analysis of a biological sample. The general workflow involves preparing tubes that contain the samples, loading them into a centrifuge, and setting the appropriate speed and time. The centrifuge then spins the samples, causing separation of at least a part of the agents. After the centrifugation, the samples are carefully removed, and a supernatant is collected for further analysis. Internal experiences with this workflow show that a biological sample may still be contaminated. Improvements are therefore desired.

### Summary

An object of the embodiments of the present disclosure is to improve an automated analysis of biological samples.

This object is solved by the disclosed embodiments, which are defined in particular by the subject matter of the independent claims. The dependent claims provide information for further embodiments. Various aspects and embodiments of these aspects are also disclosed in the summary and description below, which provide additional features and advantages.

A first aspect of the present disclosure is related to a method for processing a biological sample with an automated laboratory system,
*comprising the steps:*
- *loading a primary tube containing a primary biological sample comprising a pre-defined agent, e.g. Platelets;*
- *performing a centrifugation of the primary biological sample;*
- *transferring at least a part of the primary biological sample* as a *secondary biological sample into a secondary tube ;*
- *performing a centrifugation of the secondary biological sample* ;
- *providing a sample based on the centrifuged secondary biological sample.*

A primary tube can be any tube or container in which a liquid sample can be stored or kept. It can be a sample which may have been aliquoted from a prior tube and/or from any other container. Therefore, a primary sample may only be termed "primary" in the context of a method according to the first aspect.

A primary biological sample can comprise whole blood or other body liquids and/or may be a sample taken after some preliminary handling and/or after a first processing had been carried out. A biological sample may be any liquid that can be polluted with an agent.

An agent can be any material in a biological sample, either a natural material, such a platelets, or artificially material. An agent may refer to any substance, organism, or factor that can contaminate a biological sample. An agent can comprise a biological agent, e.g. Comprising microorganisms, cells, proteins, and/or nucleic acids. Additionally or alternatively, an agent can comprise a chemical agent, e.g. Comprising metabolites, toxins and/or drugs. Additionally or alternatively, an agent can comprise a physical agent, e.g. Nanoparticles and/or tracer elements. Additionally or alternatively, an agent can comprise an environmental agent, e.g. Pollutants and/or allergens. Additionally or alternatively, an agent can comprise a synthetic agent e.g. A diagnostic probe and/or one or more reagents.

The primary biological sample is centrifugated, in particular in the primary tube. Thereby, one or more agents in the sample may gather at a certain part of the tube. For example, if the agent is or comprises platelets, the platelets can gather at the bottom of the tube when centrifuged.

Afterwards, at least a part of the primary biological sample is transferred or aliquoted as a secondary biological sample. The transferring is performed into the secondary tube. The secondary tube can be of the same type as the primary tube. Alternatively, the secondary tube can be of a different type. For example, the secondary tube can be of a smaller type, if only a part of the primary biological sample is aliquoted into the secondary tube.

In a specific embodiment a plurality of secondary tubes can be filled with a biological sample from a centrifuged primary tube. This can be in particular the case if different tests should be applied on the centrifuged biological sample of the primary tube. Each test can be applied to a separate secondary biological sample. Additionally or alternatively, this can be the case if the biological sample of a primary tube should be centrifuged at different locations or by different methods, e.g. By different machines, in particular with different parameters for each second centrifugation.

A secondary biological sample can be centrifuged in the secondary tube. Additionally or alternatively, the secondary biological sample can be centrifuged in a different tube or container. The second centrifugation can be of the same type as the first centrifugation and/or configured in the same way as a first centrifugation. In particular the second centrifugation can be of the same duration as the first centrifugation. Additionally or alternatively, the second centrifugation can be performed with the same centrifugation trajectory, i.e. The same acceleration and rotation velocity.

However, the second centrifugation can also differ from a first centrifugation in terms of duration, acceleration and/or rotation velocity. In particular the second centrifugation can be longer and/or with higher velocity in order to remove as much of one or more agents within the biological sample as possible.

The parameters of a centrifugation can also be dependent on a test to be performed based on a biological sample from the secondary biological sample. Some tests may require a more purified biological sample and others not. It should be stressed, that a variety of different tests may be applied depending on the task at hand. Some (not all) of these tests require that agents be reliably removed down to a pre-defined concentration from the biological sample prior to the test.

During a method according to the first aspect, a biological sample may be cleaned of one or more agents, by being cleaned by double centrifugation and intermittent aliquoting. The concentration of agents (for example platelets) before centrifugation may be higher than the concentration of agents after centrifugation. A concentration of agents in a resulting liquid may be lower. Specific tests can be applied to an agent-poor biological sample, e.g. An agent-poor plasma.

After centrifugation, an agent, e.g. Platelets, may gather at the bottom and/or the side of the tube. In order to obtain a less concentrated biological sample after centrifugation, a pipet may be advanced to the upper part of the tube where no agents are concentrated. From this position the agent-poor biological sample can be transferred to a secondary tube.

A biological sample which is provided after the second centrifugation may be the whole secondary biological sample (without the agent gathered at the bottom of the tube after centrifugation) or may be based on the secondary biological sample, i.e. Maybe only a part of the secondary biological sample or a further processed biological sample comprising (a part of) the centrifuged secondary biological sample. It should be pointed out that there may exist a certain "dead volume" i.e. A volume that cannot be reached by the pipet of the aliquoter or of an analyzer, and therefore cannot be extracted.

The secondary tube can be screw-capped before centrifugation. After aliquoting, the container of the second sample, e.g. The secondary tube can be screw-capped, for centrifugation and/or for sendout. The secondary tube can remain open if it is directly provided to an analyzer, i.e. Depending on a pre-defined process.

A method according to the first aspect can provide an automated way to reduce a concentration of an agent in a biological sample in order to prepare a biological sample for testing.

An embodiment of the first aspect is related to a method for processing a biological sample with an automated laboratory system,
*wherein the primary and*/*or the secondary biological sample comprises one or more of:*
- *plasma,*
- *serum,*
- *spinal fluid,*
- *urine,*
- *stool suspension, or*
- *saliva.*

Different kinds of biological samples can be contaminated with different kinds of agents and therefore treated differently. Biological samples can in particular be lipemic samples. For example, serum, plasma, and/or cerebrospinal fluids can be contaminated with lipids. Thereby, in general, a contamination can be a result of a deliberately added agent.

An embodiment of the first aspect is related to a method for processing a biological sample with an automated laboratory system,
*comprising the step:*
- *receiving a selection information of a user and*/*or from the primary tube related to the processing of the primary tube and*/*or the primary biological sample in the primary tube.*

Selection information may be derived from various sources. For example, a user may have made a corresponding selection at the beginning of the method. It is also possible, that the analysis of the first biological sample using an evaluation algorithm has led to a decision made by the algorithm that a certain "selection" is to be carried out.

An indication that a biological sample of the (primary) tube should be centrifuged twice may be provided by a user interface. This may be based on a check-request provided to a user at a graphical user interface. Additionally or alternatively such an information may be information from the tube. This information may be determined based on a visual indication, i.e. A bar code, attached to the tube, etc. This information may be determined in a method step before the primary tube is loaded.

Selection information may be obtained regarding how the primary tube and/or a primary biological sample, respectively, should be processed. A presence of an aliquoter may indicate that an aliquoting to a secondary tube should be performed.

These examples can facilitate automated recognition of processing requirements.

An embodiment of the first aspect is related to a method for processing a biological sample with an automated laboratory system,
*comprising the step:*
- *receiving information related to the transferring of the primary biological sample into the secondary tube, in particular related to a volume.*

This information can be dependent on how the primary tube, or the primary biological sample should be processed. The information can comprise an environmental temperature, a temperature of a biological sample, a resting time between to centrifugation steps, a volume to be extracted from the centrifuged primary biological sample. Additionally or alternatively, a test, e.g. A plasma test, that should be carried out on the biological sample, may govern the transfer of the primary biological sample or a part thereof to a secondary tube.

The information can be obtained in the same way as the selection information, which is described in the context of the preceding embodiment, e.g. Via a sticker on a tube and/or a user input.

In one embodiment, the aliquoting can be performed at room temperature, the volume transferred can depend on the requested tests and/or how much transferrable volume is available in the tube after centrifugation.

These examples can facilitate a sample-dependent transferring/aliquoting.

An embodiment of the first aspect is related to a method for processing a biological sample with an automated laboratory system,
*comprising the step:*
- *receiving information related to a test, e.g. A coagulation test, of the biological sample within the primary and*/*or secondary tube.*

A test can be a coagulation test. A coagulation tests can assess an ability of a biological sample to clot, in particular blood plasma. A coagulation test in medical diagnostics can, e.g., comprise a testing for prothrombin time, activated partial thromboplastin time, international normalized ratio, d-dimer, fibrinogen level, and platelet count. These tests may evaluate blood clotting ability, diagnose bleeding disorders, monitor anticoagulant therapy, and/or assess hemostatic functions. In the case of blood-related tests, e.g. Blood coagulation tests, platelets may interfere with the results of the test. Therefore they need to be removed.

Some tests require only one centrifugation; some others require two centrifugations. Therefore, the method can comprise a workflow in which samples which are intended for two-centrifugation tests are directed to two centrifugations, and the samples which are intended for one centrifugation are directed to receive only a single centrifugation.

Some tests do not require platelets-poor plasma for these tests, platelet counts up to 200.000 /µl can be accepted. Platelets-poor plasma may be defined as plasma that contains less than 10000 platelets/µl. In particular cases, there exist centrifugation-dependent test requirements. Especially, some sensitive tests may require a specific way of centrifuging, a specific centrifuging protocol.

Many tests may require an agent-poor biological sample, e.g. Platelets-poor plasma, and therefore may receive a biological sample processed with two centrifugations. An agent-poor biological sample, e.g. a platelets-poor plasma may also be required in cases when a sample is to be frozen. Platelets-poor plasma can be achieved by low-speed centrifugation for a long time and/or by double centrifugation with an aliquotation step in between. Low speed may refer to 1000 - 2500 revolutions per minute (rpm). High speed may refer to speeds over 3000 rpm. For other centrifuges low-speed can be as low as 300 rpm, while a high-speed centrifuge could spin up to 15000 rpm. Ultracentrifuges can spin in excess of 150,000 rpm.

Another interfering agent that can be removed from a biological sample is lipids in lipemic samples. To this end, the supernatant of the sample may be transferred into a new tube and centrifuged at room temperature for, e.g., 15 min at about 10 000 g. In diagnostics, a supernatant is the clear liquid that remains above the solid or precipitated material after centrifugation or settling of a sample. It often contains dissolved substances or suspended particles and is used for further analysis to identify or quantify various components, such as proteins, metabolites, or nucleic acids.

Additionally or alternatively, lipids can be removed by an additional centrifugation (which can be a second or a third centrifugation) with an additional agent.

These examples can facilitate a selection of a test-dependent centrifugation procedure.

An embodiment of the first aspect is related to a method for processing a biological sample with an automated laboratory system,
*comprising the step:*
- *receiving an information related to:*
   - *analyzing the secondary biological sample ; and*/*or*
   -- *providing a third sample of the secondary biological sample.*

A part extracted from a sample can also be referred to as "aliquot". An aliquot can be any part, e.g. A supernatant of the centrifuged primary sample, taken out of the original sample. Its volume ratio with respect to the original volume may not be specified. On the other hand, the term "aliquot" can mean a separation into several pre-defined volumes. In particular, partial volumes can be equal among each other to the best extent possible. Alternatively, a separation into several equal masses (instead of volumes) may also be considered "aliquoting".

A first aliquot, i.e. The secondary biological sample taken from the first biological sample, can be centrifuged again before being provided to an analyzer. Additionally or alternatively, a first aliquot can be analyzed before being centrifuged for a second time.

A second aliquot, i.e. A transferring of at least a part of a centrifuged secondary biological sample, can be provided for storing and/or freezing at least a part of the sample. Additionally, a second aliquot can be provided in case at least a part of the centrifuged sample should be further processed (e.g. Analyzed) off-premises and therefore provided to a sendout region.

These examples can facilitate a decision if a sample is to be analyzed and/or aliquoted for a further time.

An embodiment of the first aspect is related to a method for processing a biological sample with an automated laboratory system,
*comprising the step:*
   - *after the second centrifugation, analyzing the secondary biological sample of the secondary tube;*
*and in particular the step:*
   - *screw-capping the secondary tube after the analysis.*

In this case no capping, in particular no screw-capping, may be necessary between an end of the centrifugation and a subsequent analysis.

A screw-capped secondary tube or a screw capped tube with a centrifuged secondary biological sample, can be stored, frozen, and/or provided to a sendout region. These examples may prepare a sample for sending out or storage.

An embodiment of the first aspect is related to a method for processing a biological sample with an automated laboratory system,
*comprising the step:*
   - *transferring at least a part of the secondary biological sample as a third*
*sample into a third tube.*

This step can be in particular implemented if a part of a secondary biological sample shall be analyzed. Additionally or alternatively, this step can be implemented, if the whole biological sample or a part of the secondary biological sample shall be stored, frozen, and/or provided to a sendout region.

These examples can facilitate analyzing, sending-out and/or storage of a biological sample, in particular after the steps specified in the two preceding embodiments.

An embodiment of the first aspect is related to a method for processing a biological sample with an automated laboratory system,
*comprising the step:*
- *screw-capping the third tube.*

In case a centrifuged secondary biological sample is provided into a third tube, the third tube can be screw-capped. A screw-cap can be a cap which can be easily applied or removed. This maybe in particular useful if a further processing is not decided or cannot be performed yet. In this case, the tube can be screw-capped and thereby secured from external influences, however, if necessary, the cap can be removed again and the sample or a part of the sample can be transferred to another tube or similar. Of course the same applies in case of screw-capping a secondary tube.

An embodiment of the first aspect is related to a method for processing a biological sample with an automated laboratory system,
*comprising the step:*
- *transferring the third tube to a pre-defined destination* .

A destination can be a long-term storage and/or a freezer. Additionally or alternatively, a pre-defined destination can be another laboratory, in particular with specialized analyzing techniques for further analyzing at least a part of the sample in the third tube.

These examples can facilitate a further processing of a second aliquot.

An embodiment of the first aspect is related to a method for processing a biological sample with an automated laboratory system,
*comprising the step:*
- *receiving information if a biological sample requires a double centrifugation.*

As already described above, a double centrifugation can be necessary and/or suggested for certain tests, e.g. Certain coagulation tests, lipemic tests, that require an agent-poor sample, i.e. Agent-poor sample.

Additionally or alternatively double centrifugation might, for example, be advantageous in case that the first centrifugation didn't allow for a sufficient separating of agent-related particles from a plasma probe. Additionally or alternatively, a double centrifugation might be advantageous in cases, where an intermediate treatment with any agent added to the sample after the first centrifugation has led to coagulation, such that new particles/agents (usually of a different type) have been created, which also have to be filtered out by centrifugation.

Based on a first centrifugation, basic tests can be carried out on a first level analysis. Based on a second centrifugation, one or more specific test, which requires a less agent-polluted sample, can be carried out as second level analysis. In a particular case, a third, fourth, or even further centrifugation steps may be necessary in order to arrive at a sample with an amount of a polluting agent that is lesser than a defined threshold.

By these examples, a double centrifugation or multi-centrifugation can be integrated into a professional contingent laboratory workflow.

An embodiment of the first aspect is related to a method for processing a biological sample with an automated laboratory system,
*comprising the step:*
- *receiving information if a biological sample requires a second centrifugation in a different tube.*

Some samples may not need a second centrifugation or at least a second centrifugation can be performed in the same primary tube as the first centrifugation. These samples can be excluded from the second centrifugation in a secondary tube. This can be implemented in an automatic procedure as part of a method according to the first aspect. This can facilitate an automatic processing of a plurality of biological samples with different requirements.

An embodiment of the first aspect is related to a method for processing a biological sample with an automated laboratory system,
*comprising the step:*
- *obtaining an information that an aliquoting means is available or not available for transferring a biological sample from one tube into another tube.*

The wording "obtaining" can mean determining by the system or receiving/fetching the information, e.g. From a system monitoring an availability of an aliquoting system.

Tubes that should be aliquoted, but presently cannot be aliquoted due to unavailability of an aliquoter, can be flagged for users and/or the method to note. If an aliquoter is not available, a tube can be stored until an aliquoter becomes available and/or sent to another aliquoting means.

An advantage of this embodiment is that in times, where no means for aliquoting is available or functioning, a sample/tube can be provided to a sendout region or centrifuged again by a different device and/or manually.

An embodiment of the first aspect is related to a method for processing a biological sample with an automated laboratory system,
*if an aliquoting means is not available for transferring a biological sample from one tube to another tube, comprising one or more of the steps:*
- *storing the tube;*
- *waiting a pre-defined time before a check is performed if the aliquoter is available again;*
- *indicating the tube for manual processing;*
- *providing the tube to a sendout region.*

In case that an aliquoting means is not available for transferring a biological sample from one tube to another tube, the automatic analyzing of the sample may need to come to a hold, at least temporarily. In particular, this can be indicated by the laboratory system. In case a sample cannot be aliquoted automatically, it may be stored to be analyzed later or be sendout to be analyzed manually. Manual processing can comprise a manual aliquoting and/or a manual transfer to another aliquoter.

In particular, a system that is not able to provide a scheduled aliquotation may be configured to wait a pre-determined time during which it tries automatic aliquotation of the tube again. After the pre-determined time, the system may then initiate a storage, a sendout (e.g. To another aliquotation facility), or signal for a manual aliquotation.

This may facilitate that a necessary or suggested first, second, or further centrifugation will not be missed.

A second aspect of the present disclosure is related to a device for preparing a biological sample with an automated laboratory system,
*configured to:*
- *load a first tube containing a first biological sample with an agent;*
- *perform a centrifugation of the first tube;*
- *transfer at least a part of the first biological sample* as a *second biological sample into a second tube ;*
- *perform a centrifugation of the second biological sample in the second tube;*
- *prepare a sample based on the second biological sample, for further aliquoting, analyzing, storing, and*/*or for sending-out.*

After a second centrifugation a biological sample can be provided that has less concentration of an agent as before a first centrifugation. Alternatively, after a second centrifugation a concentration of an agent within the biological is less than a pre-defined threshold. The laboratory system can be configured to execute a method according to the first aspect of this disclosure. The features or functions mentioned in the context of the first aspect of this disclosure can be implemented as configuration of an automated laboratory system according to the second aspect of this disclosure.

### Brief description of the figures

Further advantages and features result from the following embodiments, some of which refer to the figures. The figures do not always show the embodiments to scale. The dimensions of the various features may be enlarged or reduced, in particular for clarity of description. For this purpose the figures are at least partially schematized.
Fig. 1 illustrates a process of an automatic laboratory system according to embodiments of this disclosure.
Fig. 2 illustrates an automatic laboratory system according to embodiments of this disclosure.

In the following description reference is made to the accompanying figures which form part of the disclosure, and which illustrate specific aspects in which the present disclosure can be understood. Identical reference signs refer to identical or at least functionally or structurally similar features.

In general, a disclosure of a described method also applies to a corresponding device (or apparatus) for carrying out the method or a corresponding system comprising one or more devices and vice versa. For example, if a specific method step is described, a corresponding device may include a feature to perform the described method step, even if that feature is not explicitly described or represented in the figure. On the other hand, if, for example, a specific device is described on the basis of functional units, a corresponding method may include one or more steps to perform the described functionality, even if such steps are not explicitly described or represented in the figures. Similarly, a system can be provided with corresponding device features or with features to perform a particular method step. The features of the various exemplary aspects and embodiments described above or below may be combined unless expressly stated otherwise.

### Detailed description

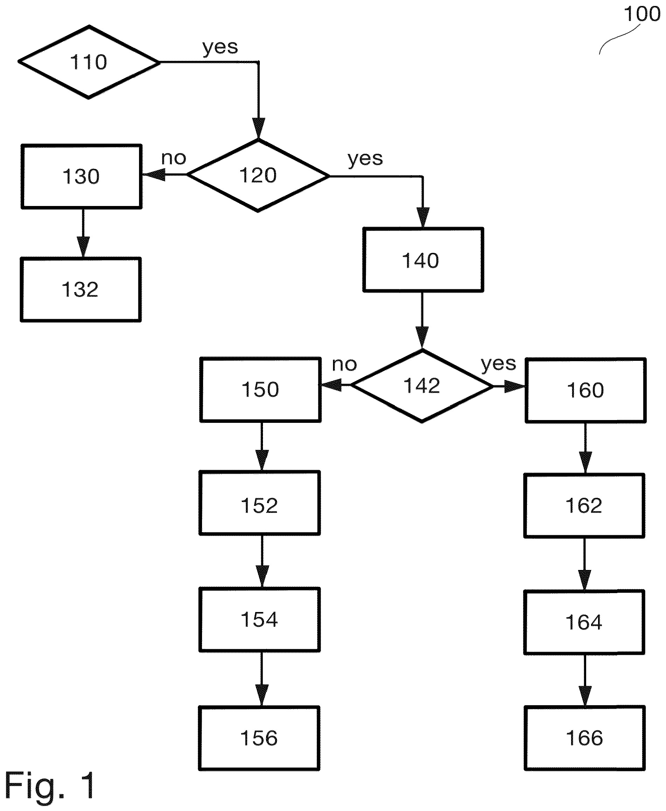

Fig. 1 illustrates a process of an automated laboratory system according to embodiments of this disclosure. The workflow illustrates an automated decision-based process 100 related to testing coagulation samples with different testing requirements.

Some biological samples need to be prepared as platelets-poor plasma which requires platelets in the plasma to be, e.g., below 10,000 per µl. This can be important because platelets contain coagulation factors at high concentrations, and their disintegration over time or due to freeze-thaw cycles can affect the test results. To achieve the necessary platelet concentration, repeated sequential centrifugation and aliquotation are employed.

The detailed workflow begins with loading the primary coagulation sample onto an automated laboratory system. In a first step 110 the process analyses the need of double centrifugation for a certain tube. Information for this check can come, for example, from a bar code or a OR-code on the tube and/or from a user input. The check for double centrifugation can also be performed on indirect information, e.g., it can be checked if a test is assigned to the tube that requires a platelets-poor plasma. Alternatively, barcode may only provide information about a tube identity and/or content. Then the workflow may be as follow. After the bar code is read, the automated laboratory system queries a database that contains information if one or more tests assigned to the tube content require a double centrifugation.

If the decision 110 for double centrifugation is positive, the automated laboratory system can assign a coagulation test to an aliquot (secondary) tube or to a first tube. This is decided in a second step 120.

If the decision 120 is negative, then a second centrifugation will be performed in the primary tube. That means, that the first centrifugation 130 and the second centrifugation 132 are performed in the same tube.

If the decision 120 is positive, then a second centrifugation will be performed on an aliquoted sample which is therefore transferred from the primary tube to a secondary tube. In an optional step, the aliquotation can be confirmed by a user in a step 140.

After the confirmation, a third check 142 is performed to decide if a sample should be analyzed off-premises.

In case the analysis should be performed on-premises, i.e., if the decision is 'no', an aliquot is created of the plasma in the primary tube and transferred to a secondary tube 150. The secondary tube can then be screw-capped. Afterwards the secondary tube is routed back to the internal centrifuge 152 and the sample is centrifuged for a second time 154. After the second centrifugation, the sample is provided in the secondary tube to an internal analyzer 156.

In case an analysis should be performed off-premises, the sample may be transferred to an automated aliquoter, where the required volume is moved to a secondary tube 160. This step can be in principle the same step as step 150. The aliquotation is performed such that the platelets that gathered on the bottom of the tube (due to the first centrifugation) are not transferred to the secondary tube. Thereby, the concentration of platelets in the plasma is reduced. The aliquot tube is screw-capped and routed to the centrifuge again 162 for a second centrifugation 164. After the second centrifugation, a part of the sample is then aliquoted into a third tube. This may be just a small part because another part of the sample should be analyzed on-premises. The second aliquotation into the third tube is then screw-capped and finally provided to a sendout-region 166, e.g. Another off-premises laboratory

In short, the workflow according to fig. 1 is as follows. At first, it is determined whether the sample requires double centrifugation. If "yes", the system checks if the customer has set rules to assign the intended aliquotation tests to an aliquot that require double centrifugation in different tubes. If the answer to the second check is "no", the primary sample is centrifuged twice in sequence without transfer to a secondary tube. If the answer is "yes", the system determines if the aliquot needs to be sent out and/or screw-capped. If the aliquot does not need to be sent out, it is created, screw-capped, centrifuged again, and analyzed by an on-premises analyzer. If the aliquot needs to be sent out, it is created (by aliquotation from the first tube) and screw-capped, centrifuged, routed back to the aliquoter, transferred into a third tube, which screw-capped again before being sent out, e.g. To an off-premises analyzer or storage facility.

In another non-disclosed workflow, which is similar to the workflow of fig. 1, the automated system can be configured as follows: firstly, a coagulation primary sample is loaded onto the automated laboratory system. Afterwards, tests are requested for the sample that require double centrifugation. According to rules of the automated laboratory system set by the customer, the system assigns the respective coagulation tests to an aliquot, i.e. A part of a primary sample in a secondary tube. The primary tube is centrifuged. The primary tube is going to the aliquoter and the required volume for the aliquot (including dead volumes in the tubes) is transferred to a secondary tube. The secondary tube is screw-capped, and the secondary tube is routed to the centrifuge and centrifuged again. In this stage, there are two options provided, which can be selected by a user.

In a first option, the secondary sample is analyzed on by the automated laboratory system. In this case, the secondary tube is transported open (i.e. Uncapped) to the analyzers of the system, afterwards the secondary tube goes to storage like any other tube. Therefore, it is capped. A cap can be a push cap or a screw-cap.

In a second option, the secondary sample finally needs to go to an sendout region. Then, at first, the secondary sample is re-routed to the aliquoter and a part or the whole sample is transferred to a third tube. The new sample in the third tube is screw-capped. Finally, the third tube is routed to the sendout region.

Embodiments that are directed to other biological samples, such as lipemic samples, can operate in the same way.

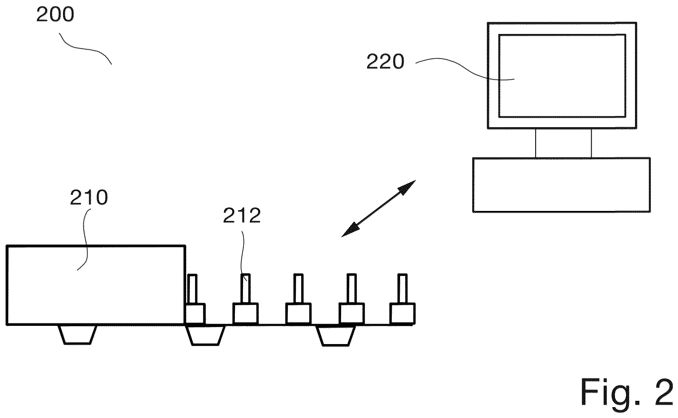

Fig. 2 illustrates an automatic laboratory system 200 configured to perform a method described in this disclosure, e.g. The method described in connection with fig. 1. The system 200 comprises an automated laboratory 210 and a computer 220. The automated laboratory 210 is configured to aliquot and analyze samples provided in tubes 212 of different types and with different requirements. The automated laboratory 210 is connected to the computer 220. The computer 220 is configured to execute at least a part of a method described herein. The computer 220 may be configured to execute a machine learning algorithm. The computer 220 and the automated laboratory 210 may be separate entities but can also be integrated together in one common housing. The computer 220 may be part of a central processing system of the automated laboratory 210 and/or the computer 220 may be part of a subcomponent of the automated laboratory 210, such as a sensor, an actor, a camera, an articulated arm, an aliquotation unit, an analyzing unit etc. Of the automated laboratory 210.

The computer 220 may be a local computer device (e.g. personal computer, laptop, tablet computer or mobile phone) with one or more processors and one or more storage devices or may be a distributed computer system (e.g. A cloud computing system with one or more processors and one or more storage devices distributed at various locations, for example, at a local client and/or one or more remote server farms and/or data centers). The computer 220 may comprise any circuit or combination of circuits. In one embodiment, the computer 220 may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (dsp), multiple core processor, a field programmable gate array (FPGA), any other type of processor or processing circuit.

A computer 220 configured to execute a method according to the present disclosure can be a network and/or cloud computer. In particular, such a computer can control a plurality of automated laboratory systems concurrently. In this way a computer can be configured to organize a transfer of a sample/tube between different automated laboratory systems that the computer controls or at least is configured to communicate with. For example, in case an aliquoter of a first automated laboratory controlled by a computer is not available, one or more samples scheduled for aliquotation at this facility can be sent to a second automated laboratory that is also controlled by this computer. Process data, e.g. A required configuration for an aliquotation, centrifugation, and/or testing of the transferred sample(s)/tube(s) are already known by the computer and may just be assigned to the second automated laboratory.

As used herein the term "and/or" includes any and all combinations of one or more of the associated listed items and may be abbreviated as "/". Expressions as, "for example", "e.g.", or "in particular" denote facultative or optional features that can be combined with all other (mandatory, facultative, or optional) features of the embodiments of this disclosure until explicitly stated otherwise.

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

### List of reference signs

- 100: automated process
- 110: check if double centrifugation required
- 120: check for aliquotation
- 130: first centrifugation
- 132: second centrifugation in first tube
- 140: confirmation of aliquotation
- 142: check for provision to sendout region
- 150: creation of aliquot
- 152: routing to centrifuge
- 154: second centrifugation
- 156: provision of sample to analyzer
- 160: creation of aliquot
- 162: routing to centrifuge
- 164: second centrifugation
- 166: provision of third tube to sendout region
- 200: automated laboratory system
- 210: automated laboratory
- 212: sample in tube
- 220: computer

## Claims

1. A method for processing a biological sample with an automated laboratory system,
comprising the steps:
- loading a primary tube containing a primary biological sample comprising a pre-defined agent, e.g. Platelets;
- performing a centrifugation (140) of the primary biological sample;
- transferring (150, 160) at least a part of the primary biological sample as a secondary biological sample into a secondary tube (140);
- performing a centrifugation of the secondary biological sample (152, 162);
- providing a sample (150) based on the centrifuged secondary biological sample.

2. The method according to the preceding claim,
wherein the primary and/or the secondary biological sample comprises one or more of:
- plasma,
- serum,
- spinal fluid,
- urine,
- stool suspension, or
- saliva.

3. The method according to the preceding claim,
comprising the step:
- receiving a selection information (120) of a user and/or from the primary tube related to the processing of the primary tube and/or the primary biological sample in the primary tube.

4. The method according to one of the preceding claims,
comprising the step:
- receiving information related to the transferring of the primary biological sample into the secondary tube, in particular related to a volume.

5. The method according to one of the preceding claims,
comprising the step:
- receiving information related to a test, e.g. A coagulation test, of the biological sample within the primary and/or secondary tube.

6. The method according to one of the preceding claims,
comprising the step:
- receiving an information (142) related to:
-- analyzing the secondary biological sample (154); and/or
-- providing a third sample (164) of the secondary biological sample.

7. The method according to one of the preceding claims,
comprising the step:
- after the second centrifugation, analyzing the secondary biological sample (154) of the secondary tube;
and in particular the step:
- screw-capping the secondary tube after the analysis.

8. The method according to one of the preceding claims,
comprising the step:
- transferring at least a part of the secondary biological sample (164) as a third sample into a third tube.

9. The method according to the preceding claim,
comprising the step:
- screw-capping the third tube.

10. The method according to one of the two preceding claims,
comprising the step:
- transferring the third tube to a pre-defined destination (166).

11. The method according to one of the preceding claims,
comprising the step:
- receiving information (110) if a biological sample requires a double centrifugation.

12. The method according to one of the preceding claims,
comprising the step:
- receiving information (120) if a biological sample requires a second centrifugation in a different tube.

13. The method according to one of the preceding claims,
comprising the step:
- obtaining an information that an aliquoting means is available or not available for transferring a biological sample from one tube into another tube.

14. The method according to one of the preceding claims,
if an aliquoting means is not available for transferring a biological sample from one tube to another tube,
comprising one or more of the steps:
- storing the tube;
- waiting a pre-defined time before a check is performed if the aliquoter is available again;
- indicating the tube for manual processing;
- providing the tube to a sendout region.

15. Automated laboratory system for processing a biological sample, configured to:
- load a first tube containing a first biological sample with an agent;
- perform a centrifugation (140) of the first tube;
- transfer (150, 160) at least a part of the first biological sample as a second biological sample into a second tube (140);
- perform a centrifugation of the second biological sample (152, 162) in the second tube;
- prepare a sample based on the second biological sample, for further aliquoting, analyzing, storing, and/or for sending-out.
